# EUROPÄISCHE PATENTSCHRIFT

(11) **EP 2 775 968 B1**
(45) Veröffentlichungstag und Bekanntmachung des Hinweises auf die Patenterteilung: **06.09.2017**
(21) Anmeldenummer: 12794864.4
(22) Anmeldetag: 12.11.2012
(51) Int. Cl.: A61F 2/962

(54) **EINRICHTUNG ZUR IMPLANTATION VON STENTELEMENTEN IN EIN HOHLORGAN**
ARRANGEMENT FOR IMPLANTING STENT ELEMENTS IN A HOLLOW ORGAN
DISPOSITIF POUR IMPLANTER DES ÉLÉMENTS D'ENDOPROTHÈSE DANS D'UN ORGANE CREUX

(30) Priorität: 11.11.2011 DE 102011118414; 11.11.2011 DE 202011107781 U; 09.11.2012 WO PCT/EP2012/004666
(43) Veröffentlichungstag der Anmeldung: 17.09.2014
(73) Patentinhaber: MediGroup GmbH, 75179 Pforzheim (DE)
(72) Erfinder: AMENDT, Klaus, 68163 Mannheim (DE); JUNG, Johannes, 75181 Pforzheim (DE); KÖLBLE, Heinz, 77955 Ettenheim (DE)
(74) Vertreter: Fritzsche, Thomas
(86) Internationale Anmeldenummer: PCT/EP2012/004696
(87) Internationale Veröffentlichungsnummer: WO 2013/068127

(56) Entgegenhaltungen:
- EP-A1- 1 894 545
- WO-A1-2006/026371
- US-A1- 2008 234 795

## Beschreibung

Die Erfindung betrifft eine Einrichtung zum Einsetzen von mindestens einem Stentelement in ein Hohlorgan umfassend einen Schaft mit einem proximalen Ende und einem eine atraumatische Spitze aufweisenden distalen Ende, wobei mehrere voneinander unabhängige Stentelemente nebeneinander beabstandet auf einer axialen Linie innerhalb des Schafts angeordnet sind und wobei der Schaft ein Expandieren der Stentelemente verhindert. Stents werden als Prothesen zur Erweiterung und Offenhaltung in röhrenförmige Hohlorgane, wie Blutgefäße, eingesetzt. Derartige Stents weisen in der Regel eine gitter- oder spiralförmige Struktur aus Materialstegen auf. Zwischen den Materialstegen sind materialfreie Bereiche gebildet, die es ermöglichen, dass diese Struktur an ihrem Implantationsort in das Gewebe einwachsen kann. Solche Stents sind beispielsweise in der DE-A 197 46 88 beschrieben.

In biegsamen Gefäßbereichen, wie beispielsweise im Knie, besteht bei Prothesen nach dem Stand der Technik das Problem, dass im Bereich der Biegung durch den Stent erhebliche Stauch-, Zug- und Drehbewegungen auftreten. Außerdem wirken dabei auch zusätzliche Reibungskräfte auf die Intima der Blutgefäße ein. Des Weiteren werden durch die hierbei entstehenden oben genannten Stauch-, Zug- und Drehbewegungen die Materialstege geschwächt, so dass es zu Brüchen und zu einzelnen Ablösungen von Stent-Fragmenten kommen kann. Dies wiederum kann zu Verletzungen der Gefäßwand, einer Restenose, z. B. durch Narbenbildung an der Gefäßwand, oder sogar zu einer Ausbildung eines Aneurysmas führen. Auch Embolien können ggf. hierdurch ausgelöst werden.

In der Druckschrift DE 600 30 705 T2 wird ein Katheter zum Implantieren von Stents beschrieben, welcher eine Hülle aufweist, wobei in der Hülle einen mehrteiligen Stent vorhanden sein kann. Der mehrteilige Stent besteht aus benachbarten Stentteilen, welche über Verbindungselemente miteinander verbunden sind. Bei Einsetzen des Stents in eine Arterie wird das Verbindungselement ggf. durch eine Expansion des Stentteils physikalisch von einem benachbarten Stentteil getrennt. Jedoch ist hierbei ein exaktes Legen bzw. Platzieren des einzelnen Stentteils nahezu unmöglich, da ein solches physikalische Trennen der Stentteile nicht immer uniform verläuft. Hinzu kommt der Nachteil, dass Reste der Verbindungselemente an den platzierten Stentteilen verbleiben, welche wiederum zu einer Verletzung der Gefäßwand beitragen können.

US 2008/0234795 A1 beschreibt einen Katheter zur Einbringung einer Prothese in ein Hohlorgan. Dabei umfasst der Katheter einen ausgedehnten flexiblen Teil und einen darüber verschiebbaren angeordneten Mantelpunkt. Weiter umfasst der Katheter eine Vielzahl von selbst expandierenden tubulären Prothesen, welche entlang des ausgedehnten flexiblen Teils axial-beabstandet innerhalb des Mantels angeordnet sind. Die Prothesen können dabei wahlweise ineinandergreifend angeordnet sein. Ebenfalls weist der Katheter eine Art Schieber (Englisch "resilient fingers") auf, welcher bewegbar entlang des ausgedehnten flexiblen Teils innerhalb des Mantels angeordnet ist. Der Schieber ist dabei derart ausgebildet, dass bei einer Bewegung in proximaler Richtung die Prothesenelemente in ihrer Lage innerhalb des Mantels unberührt bleiben, jedoch bei einer Bewegung in distaler Richtung die Prothesenelemente verschoben werden können, so dass diese eine feste Verbindung durch Ineinandergreifen von Verbindungselementen ausbilden können. Hierbei ist jedoch zu beachten, dass die Verbindungselemente ein Teil der gesamten Prothese sind.

EP 1 894 545 A1 beschreibt eine Anordnung von Stents auf einem Schaft. Dabei sind die Stents jedoch lose auf dem Schaft angeordnet und können daher aus der äußeren Hülle vorzeitig herausrutschen, sobald diese zurückgezogen wird.

WO 2006/026371 A1 betrifft ein System zur Verabreichung bzw. zum Platzieren von einer Vielzahl von unterschiedlichen medizinischen Objekten, wie beispielsweise Venenklappen, welche sich beim Freisetzen im Blutgefäß expandieren. Diese Objekte bzw. Komponenten liegen jedoch nicht fest verbunden mit dem inneren Schaft vor.

Eine Aufgabe der Erfindung besteht darin, eine Einrichtung zum Einsetzen von mindestens einem Stentelement in ein Hohlorgan zur Verfügung zu stellen, so dass unter anderem bei einer Implantation in biegsamen Gefäßen die beschriebenen Nachteile vermieden werden. Desweiteren soll eine Einrichtung bereitgestellt werden, die es einem Arzt ermöglicht, mittels eines einzigen Implantationsbestecks bzw. Katheters an verschiedenen Körperstellen Stents einzusetzen.

Die Aufgabe wird durch eine Einrichtung zum Einsetzen von Stentelementen in ein Hohlorgan gemäß Anspruch 1 gelöst. In den Unteransprüchen sind vorteilhafte Weiterbildungen und bevorzugte Ausführungsformen angegeben.

Die erfindungsgemäße Einrichtung ist zum Einsetzen oder zur Implantation von mindestens einem Stentelement in einem Hohlorgan, insbesondere einem Blutgefäß vorgesehen. Statt eines einzelnen mehr oder weniger biegsamen, länglich röhrenförmigen Stents wird vorgeschlagen, die erfindungsgemäße Einrichtung aus einer Mehrzahl von einander unabhängiger Stentelementen zu bilden, insbesondere von kurzen Stentelementen, welche auf einer axialen Linie angeordnet sind, wobei zwischen benachbarten Elementen jeweils ein Abstand derart gebildet ist, dass die Elemente problemlos einzeln und zeitlich unabhängig von einander eingesetzt bzw. implantiert werden können. Durch das Implantieren von mehreren einzelnen, nicht miteinander verbundenen Stents, die dadurch unabhängig von einander frei beweglich sind, wird eine Beanspruchung durch Reibung an der Innenseite der Gefäße vermieden. Darüber hinaus ist es möglich, Stents mittels eines einzigen Katheters/Bestecks an völlig verschiedenen Stellen im Körper zu applizieren. Hierdurch wird nicht nur die Belastung eines Patienten verringert, sondern dies führt auch zu einer beachtlichen Kosteneinsparung.

Frei beweglich im Sinne der Erfindung sind die Stentelemente dadurch, dass diese nach der Implantation nicht untereinander verbunden sind, also lediglich relativ zueinander frei beweglich sind, während die vorgesehene Verbindung der Stents mit dem Gewebe der Gefäßwand natürlich hergestellt wird. Dadurch wird vorteilhaft die Reibung mit der Gefäßwand bei einem Biegen des Gefäßes reduziert. Die Innenseite der Gefäßinnenwand verläuft auf Grund der Stauchung bei der Biegung meist wellenförmig. Bei der erfindungsgemäßen Einrichtung besteht vorteilhaft die Möglichkeit, dass diese gestauchten Gefäßbereiche in die Zwischenräume zwischen den Stentelementen eindringen, ohne dass hier zusätzliche Reizungen entstehen. Ebenso wird durch die von einander unabhängigen Stentelemente eine unmittelbare Kraftübertragung unterbunden, wodurch Spannungen in den durch materialfreie Bereiche und Materialstege definierten Mantelflächen der Stentelemente vermieden werden.

Die axiale Linie, auf welcher die Stentelemente angeordnet sind, wird bei der Implantation innerhalb des Schaftes in das Gefäß eingeführt. Die Mantelflächen der röhrenförmigen Elemente umgeben die axiale Linie in radialem Abstand. Vorzugsweise sind die Stentelemente selbstexpandierend bzw. selbstexpandierbar und weisen vor der Implantation eine geringere radiale Ausdehnung auf und expandieren während bzw. nach der Implantation auf eine größere radiale Ausdehnung. Vorteilhaft ist die axiale Linie elastisch, insbesondere biegeelastisch.

Die erfindungsgemäß vorgesehenen Stentelemente sind insbesondere einstückig ausgebildet, vorzugsweise aus Metall, wobei die Stentelemente besonders bevorzugt aus einem einstückigen Metallrohr geschnitten sind. Das Schneiden kann insbesondere mit Lasern oder anderen geeigneten, hochpräzise steuerbaren Schneidwerkzeugen erfolgen. Als Schneiden im Sinne der Erfindung wird dabei jedes geeignete Verfahren zur Erzeugung der erfindungsgemäßen Strukturen verstanden. Das Schnittmuster ist an sich beliebig wählbar. Vorzugsweise sind jedoch elastische, insbesondere biegeelastische Schnittmuster bevorzugt. Allerdings ist ein einfaches rautenförmiges Schnittmuster bevorzugt.

Bei der erfindungsgemäßen Einrichtung sind die einzelnen Teile der Stentelemente vorzugsweise vor ihrer Implantation auf bzw. in der Einrichtung miteinander sicher und ggf. mittels Abstandshalter miteinander verbunden, und haften nicht nur an einer oder wenigen Stellen aneinander. Die Stentelemente können aus beliebigem Material bestehen, solange es für den Gebrauch im menschlichen Körper geeignet ist und solange es die für einen Stent notwendigen Eigenschaften aufweist, wie z. B. Elastizität und ausreichende Stützfunktion. Typische Materialien sind insbesondere Edelstahl oder eine Nickel-Titan-Legierung, vorzugsweise eine Nitinol- oder eine andere Formgedächtnislegierung. Die Oberfläche ist ggf. elektroplattiert und/oder elektropoliert oder auf andere Weise veredelt, um eine hinreichend glatte und verträgliche Oberfläche zu gewährleisten. Weiterhin bevorzugt sind die Stentelemente röntgenopak ausgestaltet oder weisen zumindest röntgenopake Markierungen auf, welche es dem behandelnden Arzt ermöglichen, beim Setzen der Stentelemente diese so genau wie möglich zu positionieren, da die Lage jedes einzelnen Stentelements dadurch sichtbar gemacht werden kann. Dies ermöglicht auch eine spätere Lagekontrolle der Stentelemente bei nachfolgenden medizinischen Untersuchungen.

Die Mantelflächen der Stentelemente sind zweckmäßigerweise zylindrisch. Ggf. weisen die Elemente an nur einem oder an beiden axialen Enden eine Abschrägung auf. Allgemein ist gemäß einer bevorzugten Ausführungsform vorgesehen, dass eine Höhe der Mantelfläche mindestens eines Stentelements über einen Umfang der Mantelfläche variiert. Dadurch kann derjenige Bereich der Mantelfläche, welcher eine verkürzte Höhe aufweist, im Bereich der Innenkrümmung des gebogenen Gefäßes angeordnet werden, wodurch die Zwischenräume, in welche die bei Beugung gestauchte Gefäßwand eindringen kann, vergrößert werden. Bevorzugt ist dazu ein Maximum der Höhe der Mantelfläche einem Minimum der Höhe der Mantelfläche auf dem Umfang der Stentelemente gegenüberliegend angeordnet, wodurch ein rautenförmiger Längsquerschnitt entsteht.

Gegenüber Prothesen nach dem Stand der Technik sind die Stentelemente der erfindungsgemäßen Einrichtung vorzugsweise derart verkürzt, dass gemäß einer bevorzugten Ausführungsform ein Verhältnis einer maximalen Länge der Mantelfläche zu einem Durchmesser bei mindestens einem Stentelement mindestens das 1,0-, insbesondere mindestens das 1,5- bzw. 1,8- bzw. 2-fache beträgt. Besonders bevorzugt sind Verhältnisse von mindestens dem 2,3-fachen. Typische Obergrenzen für das Verhältnis betragen max. das 8- bzw. 6-, insbesondere max. 5-fache, wobei das max. 4-fache bzw. 3,5-fache besonders bevorzugt ist.

Gemäß einer weiteren bevorzugten Ausführungsform der Einrichtung ist vorgesehen, dass vor der Implantation zwischen den Stentelementen Abstandhalter angeordnet sind. Dadurch wird das Einsetzen bzw. das Legen der Stentelemente vereinfacht, insbesondere da die einzelnen Elemente mit Abstand zueinander anzuordnen sind. Die Abstandhalter weisen dazu vorzugsweise, zumindest vor der Implantation, eine zu den Stentelementen komplementäre Form auf, womit zum Ausdruck gebracht wird, dass die Form der Abstandhalter bevorzugt derart auf die Form der Stentelemente abgestimmt sein soll, dass die Abstandhalter den Zwischenraum zwischen zwei benachbarten Elementen ausfüllen.

Dadurch wird durch die erfindungsgemäße Einrichtung das Legen der Stentelemente besser handhabbar, was besonders vorteilhaft ist, da jedes Stentelement nicht nur durch den Transport entlang der Gefäßbahn in seine axiale Position gebracht werden muss, sondern auch durch entsprechendes Drehen derart positioniert wird, dass dem Bereich mit verkürzter Länge der Mantelfläche der Innenseite der Biegung der Gefäßinnenwand und der Bereich mit größerer Länge der Außenseite der Biegung des Gefäßes zugeordnet wird. Ein solches Stentelement weist einen konischen oder trapezförmigen, ggf. auch einen orthogonal trapezförmigen Querschnitt auf. Allerdings können die erfindungsgemäßen Prothesen auch zylindrische Stentelemente umfassen oder auch ausschließlich daraus bestehen. Die stabilisierend wirkenden Abstandhalter erlauben ein Bewegen des jeweils zu legenden Stentelements so, als wenn nur ein längliches Stentelement zu legen wäre. Bei selbstexpandierbaren Stentelementen entspricht die komplementäre Form der Abstandhalter der Form der Stentelemente mit geringer radialer Ausdehnung, welche die Stentelemente bis zur Implantation aufweisen. Nach der Expansion auf die größere radiale Ausdehnung sind die Abstandhalter bevorzugt nicht mehr komplementär in der Form, da die Abstandhalter ihre ursprüngliche Form beibehalten und nicht expandieren.

Besonders bevorzugt ist der Schaft mit ggf. innenliegender axialer Linie mit wiederum ggf. besetzten Stentelementen und/oder Abstandhaltern nach der Implantation durch die Innenseite bzw. das Innenvolumen der Stentelemente hindurch führbar. Auf diese Weise können die Abstandhalter nach dem Legen der Stentelemente wieder entfernt werden, ggf. auch durch bereits gesetzte expandierte Stentelemente hindurch. Ggf. können so auch weitere Stentelemente vor und/oder nach dem bereits implantierten Stentelement gelegt werden. Im Sinne der Erfindung ist es auch möglich, mit der erfindungsgemäßen Einrichtung innerhalb eines bereits expandierten, platzierten Stentelements ein weiteres Stentelement abzulegen, dadurch wird die nach außen wirkende Radialkraft verdoppelt bzw. verstärkt. Ggf. können die Abstandhalter untereinander verbunden sein, z. B. auf einem Faden, einem Draht oder einer Hülse, welche als axiale Linie ausgebildet sind. Die Abstandhalter bestehen üblicherweise aus demselben Material wie die Stentelemente. Sie können jedoch auch aus einem beliebigen anderen kompatiblen Material, wie z. B. Kunststoff, bestehen. Sie können Röntgenopak als auch nicht opak ausgestaltet sein. Durch die Anordnung der Stentelemente ist es möglich, die Stentelemente so abzulegen, dass diese auch bei extrem verdrehten Beanspruchungen oder auch Läsionen jeweils optimal zu liegen kommen.

Gemäß einer weiteren bevorzugten Ausführungsform der Einrichtung ist vorgesehen, dass die Stentelemente mittels Abstandhalter als Kupplungselement bzw. Verbindungsanker lösbar verbunden sind, wodurch die Stabilität beim Legen der Stentelemente weiter erhöht wird. Besonders bevorzugt ist die jeweilige Verbindung durch ein Expandieren des Stentelements während der Implantation trennbar. Dazu ist die Verbindung insbesondere in axialer Richtung formschlüssig, nicht aber in zumindest einer radialen Richtung, so dass die Expansion des Stentelements beim Legen automatisch zu einer Trennung der Verbindung führt.

Mit der Erfindung ist auch ein Verfahren zum Einsetzen mindestens eines Stentelements in oder um ein Hohlorgan beschrieben, durch Legen einer Mehrzahl von Stentelementen, insbesondere von selbstexpandierenden Stentelementen, wobei Mantelflächen der jeweiligen Stentelemente durch materialfreie Bereiche und Materialstege definiert sind, wobei die Stentelemente auf einer axialen Linie derart angeordnet werden, dass zwischen benachbarten Stentelementen jeweils ein Abstand gebildet wird und wobei die Stentelemente nach der Implantation frei beweglich sind.

Bevorzugt werden zwischen den Stentelementen vor der Implantation Abstandhalter angeordnet. Weiterhin bevorzugt werden die Stentelemente mit den jeweils angrenzenden Abstandhaltern vor der Implantation lösbar verbunden. Weiterhin bevorzugt werden die Stentelemente durch Expandieren während der Implantation von den Abstandhaltern getrennt. Weiterhin bevorzugt werden die Abstandhalter nach der Implantation durch die Stentelemente hindurchgeführt, um die Abstandhalter vorteilhaft zu entfernen.

Nachfolgend wird die Erfindung anhand von Ausführungsbeispielen in Zeichnungen näher beschrieben. Die Erläuterungen sind lediglich beispielhaft und schränken den allgemeinen Erfindungsgedanken nicht ein.

Es zeigen
Figuren 1A und 1B expandierte Stentelemente nach der Implantation mittels der erfindungsgemäßen Einrichtung in einer schematischen Darstellung;
Figuren 2A und 2B nicht expandierte Stentelemente vor der Implantation mittels der erfindungsgemäßen Einrichtung in einer schematischen Darstellung;
Figur 3 ein Stentelement in einer perspektivischen Darstellung;
Figuren 4 und 5 mehrere von einander unabhängige Stentelemente nach der Implantation mittels der erfindungsgemäßen Einrichtung in einer schematischen Darstellung;
Figuren 6 und 7 mehrere Stentelemente sowie eine axiale Linie mit Abstandhaltern nach der Implantation mittels der erfindungsgemäßen Einrichtung in einer schematischen Darstellung; und
Figur 8 eine erfindungsgemäße Einrichtung zum Einsetzen von mindestens einem Stentelement in ein Hohlorgan in einer schematischen Darstellung.

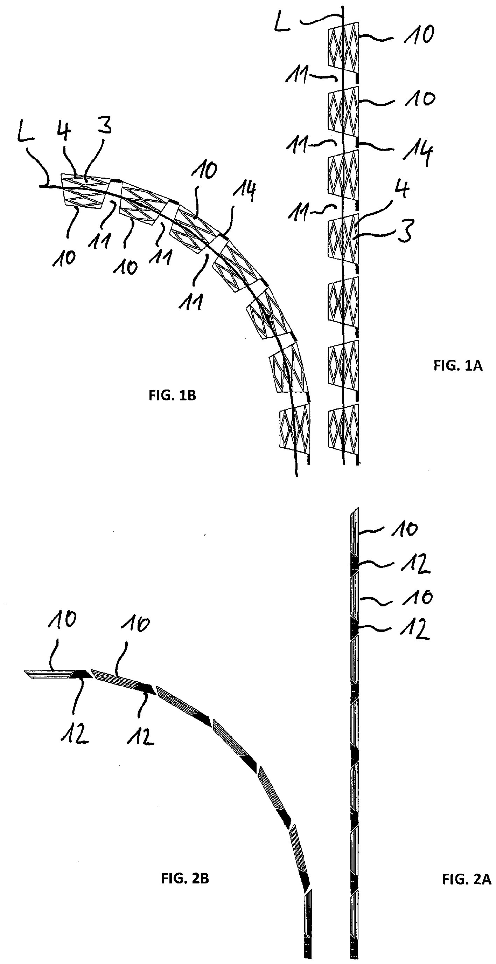

In den Figuren 1A und 1B ist jeweils eine Mehrzahl von röhrenförmigen Stentelementen 10, welche auf einer axialen Linie L angeordnet sind, wobei die Linie L in Figur 1A geradlinig verläuft und in Figur 1B gebogen ist, in einer Seitenansicht nach der Implantation mittels der erfindungsgemäßen Einrichtung dargestellt. Dies verdeutlicht, dass zwischen benachbarten Stentelementen 10 jeweils ein Abstand 11 gebildet ist, wodurch die Stentelemente nach der Implantation frei beweglich sind.

Der Vorteil der erfindungsgemäßen Einrichtung besteht darin, dass die Stentelemente 10 nach dem Legen in das Gefäß bzw. Hohlorgan nicht untereinander verbunden sind und eine wellenförmig gestauchte Gefäßinnenwand beim Beugen in die freien Abstände 11 zwischen den Stentelementen 10 hinein ausweichen kann. Dadurch wird eine Reizung des Gewebes ebenso vermieden, wie eine übermäßige Spannungsbeanspruchung der aus materialfreien Bereichen 3 und Materialstegen 4 gebildeten Struktur der Stentelemente 10. An den Stentelementen 10 sind Verbindungsanker 14 erkennbar, welche nicht mit dem jeweils benachbarten Stent verbunden sind. Aus Gründen der Übersichtlichkeit wurde lediglich eine Auswahl der sich bei jedem Stent wiederholenden Einzelheiten exemplarisch mit einem Bezugszeichen versehen.

Die in den Figuren 1A und 1B dargestellten Stentelemente 10 sind in einem Zustand nach der Implantation gezeigt, insbesondere in einem expandierten Zustand, da die Stentelemente 10 bevorzugt selbstexpandierbar sind. Der in den Figuren 2A und 2B dargestellte Zustand vor der Implantation ist im Sinne der Erfindung derjenige Zustand, den die Stentelemente 10 bis zu dem individuellen Einsetzen aufweisen. Der gelegte Stent 10 stellt somit den Zustand nach der Implantation im Sinne der Erfindung dar. Bezogen auf alle Stentelemente, welche in einer Ausführungsform der erfindungsgemäßen Einrichtung umfasst sind, kann also auch eine Teilmenge der Stentelemente 10 im Zustand vor der Implantation sein, während eine andere Teilmenge bereits in den Zustand nach der Implantation übergegangen ist.

Die in den Figuren 2A und 2B dargestellten Stentelemente zeigen alle Stentelemente 10 vor der Implantation in einem nicht expandierten Zustand, verbunden jeweils durch Abstandhalter 12, welche eine zu den Stentelementen 10 komplementäre Form aufweisen, diese also zur durchgehenden Zylinderform ergänzen. Die Stentelemente 10 weisen in der Seitenansicht eine trapezförmige Projektion auf, was durch jeweils abgeschrägte axiale Enden des röhrenförmigen Stentelements 10 hervorgerufen wird, worauf im Zusammenhang mit nachfolgend beschriebenen Figuren noch eingegangen wird.

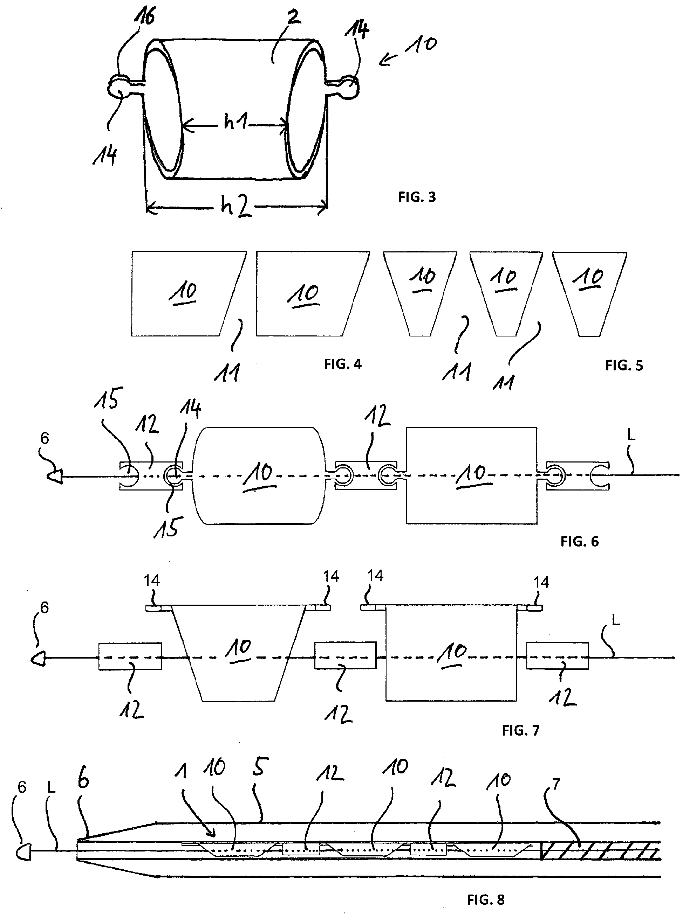

In der Figur 3 ist dazu ein einzelnes Stentelement 10 in einer perspektivischen Darstellung gezeigt, wobei die Struktur der Mantelfläche 2 hier schematisch glatt dargestellt ist. Grundsätzlich können die relativ kurzen Stentelemente die Form eines Hohlzylinders mit konstanter Höhe haben. Bevorzugt weist die Mantelfläche 2 eine über den Umfang variable Höhe auf, wobei in der Darstellung ein Minimum h1 der Höhe einem Maximum h2 auf dem Umfang gegenüberliegend angeordnet ist. Wird das Stentelement10 mit der minimalen Höhe h1 der wellenförmig gestauchten Gefäßinnenwand beim Beugen zugewandt gelegt, so stehen vorteilhaft größere Abstände 11 (siehe Figur 1B) zwischen den Stentelementen 10 zur Verfügung. Zur Verbindung mit den in Figuren 2A und 2B gezeigten Abstandhaltern 12 weist das Stentelement10 beispielsweise beidseitig einen Verbindungsanker 14 auf. Weiterhin bevorzugt ist das Stentelement 10 röntgenopak oder mit einem röntgenopaken Marker 16 versehen, welcher besonders bevorzugt auf dem Verbindungsanker 14 angebracht wird.

Die Figuren 4 und 5 verdeutlichen weitere Beispiele möglicher Formen der Stentelemente 10, wobei die grundlegende Röhrenform erhalten bleibt. Die Gestaltung der axialen Enden der Stentelemente bzw. der Höhe der Mantelfläche sind in der Seitenansicht erkennbar, ebenso die entsprechende Auswirkung auf die Abstände 11. Bei dem Beispiel gemäß Figur 4 ist lediglich jeweils ein axiales Ende der Stentelemente 10 schräg geformt, wodurch der Aufwand für die Herstellung verringert ist.

In der Figur 5 sind Stentelemente 10 dargestellt, die eine deutlich geringere Höhe der Mantelfläche in axialer Richtung aufweisen, wobei diese Höhe zusätzlich noch über den Umfang variiert, sodass das Minimum der Höhe erheblich kürzer ist, als das Maximum. Die Stentelemente 10 erhalten dadurch eine Keilform mit beinahe spitzem Zulauf.

Anhand der Figuren 6 und 7 soll die Verbindung der Stentelemente 10 mit den Abstandhaltern 12 und insbesondere deren Lösung näher erläutert werden. Die Abstandhalter 12, welche auf einer axialen Linie L angeordnet sind, weisen Hinterschnitte 15 auf, in welchen die Verbindungsanker 14 der Stentelemente 10, welche ebenfalls auf der axialen Linie L angeordnet sind, eine in axialer Richtung formschlüssige Verbindung herstellen. Gelöst wird die Verbindung durch eine radiale Relativbewegung von Stent 10 und Abstandhalter 12, beispielsweise durch die Expansion des Stentelements 10 während der Implantation. Ggf. weist in einer vorteilhaften Ausführungsform der erfindungsgemäßen Einrichtung die axiale Linie L am distalen Ende eine atraumatische Spitze 6 auf.

In der Figur 8 ist eine erfindungsgemäße Einrichtung zum Einsetzen mindestens eines Stentelements in ein Hohlorgan gezeigt, wobei die Einrichtung mindestens einen Schaft 5 mit einem proximalen Ende und einem ggf. eine atraumatische Spitze 6 aufweisenden distalen Endeumfasst und wobei mehrere voneinander unabhängige Stentelemente 10 nebeneinander beabstandet auf einer axialen Linie L innerhalb des Schafts 5 angeordnet sind, wobei die Stentelemente 10 jeweils mit Abstandhaltern 12 lösbar verbundenen sind. Der Schaft 5 verhindert ein Expandieren der Stentelemente 10 und erlaubt dadurch, dass die axiale Linie L innerhalb des Schafts 5 in axialer Richtung verschiebbar und drehbar angeordnet ist, eine genaue Positionierung der einzelnen Stentelemente 10, die erst nach dem Zurückziehen des Schaftes 5 oder einer distal axialen Verschiebung der axialen Linie L expandieren und dadurch ihre Verbindung zu den Abstandhaltern 12 unterbrechen. Die Abstandhalter 12 expandieren nicht und können vorteilhaft nach erfolgter Implantation durch die expandierten Stentelemente 10 hindurch, zurück in den Katheterschaft 5 gezogen werden, was auch in Figur 7 deutlich erkennbar ist. Ggf. weist in einer vorteilhaften Ausführungsform der erfindungsgemäßen Einrichtung die axiale Linie L am distalen Ende eine atraumatische Spitze 6 auf. In einer bevorzugten Aufführungsform der erfindungsgemäßen Einrichtung ist hinter der mit den Stentelementen 10 besetzten axialen Linie L innerhalb des Schaftes 5 ein Füllmaterial angeordnet, um ein Abknicken des Schafts 5 zu vermeiden.

### Bezugszeichenliste:

- 2: Mantelfläche
- 3: materialfreier Bereich
- 4: Materialstege
- 5: Schaft
- 6: atraumatische Spitze
- 7: Füllmaterial
- 10: Stentelement
- 11: Abstand
- 12: Abstandhalter

- 14: Verbindungsanker
- 15: Hinterschnitte
- 16: röntgenopaker Marker

- L: axiale Linie
- h1: Maximum der Höhe
- h2: Minimum der Höhe

## Patentansprüche

1. Einrichtung zum Einsetzen von mindestens einem Stentelement (10) in ein Hohlorgan, wobei die Einrichtung mindestens einen Schaft (5) mit einem proximalen Ende und einem eine atraumatische Spitze (6) aufweisenden distalen Ende umfasst und wobei mehrere voneinander unabhängige Stentelemente (10) nebeneinander beabstandet auf einem Faden, einem Draht oder einer Hülse, welche als axiale Linie (L) ausgebildet sind, innerhalb des Schafts (5) angeordnet sind, wobei der Schaft (5) ein Expandieren der Stentelemente (10) verhindert, **dadurch gekennzeichnet, dass** ein oder mehrere Abstandhalter (12) zwischen den Stentelementen (10) auf der axialen Linie (L) angeordnet sind und
die Stentelemente (10) mit den jeweils angrenzenden Abstandhalter (12) mittels einer lösbaren Verbindung verankert sind, die durch ein Expandieren des Stentelements (10) trennbar ist und
dass die Abstandshalter (12) mit der axialen Linie nicht lösbar verbunden sind.

2. Einrichtung nach Anspruch 1, **dadurch gekennzeichnet, dass** die jeweiligen Stentelemente (10) durch eine Mantelflächen (2) mit materialfreien Bereichen (3) und Materialstegen (4) definiert sind.

3. Einrichtung nach einem der vorhergehenden Ansprüche, **dadurch gekennzeichnet, dass** eine Höhe der Mantelfläche (2) mindestens eines Stentelements (10) über einen Umfang der Mantelfläche variiert.

4. Einrichtung nach einem der vorhergehenden Ansprüche, **dadurch gekennzeichnet, dass** ein Maximum der Höhe (h1) der Mantelfläche (2) einem Minimum (h2) der Höhe der Mantelfläche auf dem Umfang gegenüberliegend angeordnet ist.

5. Einrichtung nach einem der vorhergehenden Ansprüche, **dadurch gekennzeichnet, dass** mindestens ein Stentelement (10) ein Verhältnis von seiner maximalen Höhe der Mantelfläche (2) zu seinem Durchmesser zwischen 0,5 und 6 aufweist.

6. Einrichtung nach einem der vorhergehenden Ansprüche, **dadurch gekennzeichnet, dass** der/die Abstandhalter (12) eine zu den Stentelementen (10) komplementäre Form aufweisen.

7. Einrichtung nach einem der vorhergehenden Ansprüche, **dadurch gekennzeichnet, dass** innerhalb des Schafts (5) und hinter der mit den Stentelementen (10) besetzten axialen Linie (L) ein Füllmaterial (7) angeordnet ist.

## Claims

1. An arrangement for implanting at least one stent element (10) in a hollow organ, wherein the arrangement comprises at least one shaft (5) with a proximal end and a distal end having an atraumatic tip (6), and wherein several stent elements (10) independent of one another are arranged within the shaft (5) spaced apart next to one another on a thread, a wire or a sleeve, and which are formed as an axial line (L), wherein the shaft (5) prevents an expansion of the stent elements (10), **characterized in that**
one or more spacers (12) are arranged between the stent elements (10) on the axial line (L), and
the stent elements (10) with the respective adjacent spacers (12) are anchored by means of a detachable connection which can be separated by an expansion of the stent element (10), and
**in that** the spacers (12) are non-detachably connected to the axial line.

2. The arrangement according to Claim 1, **characterized in that** the respective stent elements (10) are defined by a lateral surface (2) with material-free areas (3) and material bridges (4).

3. The arrangement according to any one of the preceding claims, **characterized in that** a height of the lateral surface (2) of at least one stent element (10) varies over a periphery of the lateral surface.

4. The arrangement according to any one of the preceding claims, **characterized in that**, on the periphery, a maximum height (h1) of the lateral surface (2) is arranged opposite a minimum height (h2) of the lateral surface.

5. The arrangement according to any one of the preceding claims, **characterized in that** at least one stent element (10) has a ratio of the maximum height of the lateral surface (2) thereof to the diameter thereof between 0.5 and 6.

6. The arrangement according to any one of the preceding claims, **characterized in that** the spacer/spacers (12) has/have a shape that is complementary to the stent elements (10).

7. The arrangement according to any one of the preceding claims, **characterized in that**, within the shaft (5) and behind the axial line (L) occupied by the stent elements (10), a filling material (7) is arranged.

## Revendications

1. Dispositif pour implanter au moins un élément d'endoprothèse (10) dans un organe creux, dans lequel le dispositif comporte au moins une tige (5) dotée d'une extrémité proximale et d'une extrémité distale présentant une pointe atraumatique (6) et dans lequel plusieurs éléments d'endoprothèse indépendants les uns des autres (10) sont disposés à distance les uns des autres sur un fil, un fil métallique ou un manchon qui sont constitués comme une ligne axiale (L), à l'intérieur de la tige (5), dans lequel la tige (5) empêche un déploiement des éléments d'endoprothèse (10), **caractérisé en ce que**
un ou plusieurs espaceurs (12) sont disposés entre les éléments d'endoprothèse (10) sur la ligne axiale (L) et
les éléments d'endoprothèse (10) avec les espaceurs (12) respectivement adjacents sont ancrés au moyen d'une liaison détachable qui peut être séparée par un déploiement de l'élément d'endoprothèse (10) et
**en ce que** les espaceurs (12) sont reliés de façon non détachable avec la ligne axiale.

2. Dispositif selon la revendication 1, **caractérisé en ce que** les éléments d'endoprothèse respectifs (10) sont définis par une surface d'enveloppe (2) comportant des zones sans matériau (3) et des pattes de matériau (4).

3. Dispositif selon l'une des revendications précédentes, **caractérisé en ce qu'**une hauteur de la surface d'enveloppe (2) d'au moins un élément d'endoprothèse (10) varie sur un pourtour de la surface d'enveloppe.

4. Dispositif selon l'une des revendications précédentes, **caractérisé en ce qu'**un maximum de la hauteur (h1) de la surface d'enveloppe (2) est disposé de façon opposée à un minimum (h2) de la hauteur de la surface d'enveloppe sur le pourtour.

5. Dispositif selon l'une des revendications précédentes, **caractérisé en ce qu'**au moins un élément d'endoprothèse (10) présente un rapport de sa hauteur maximale de surface d'enveloppe (2) à son diamètre entre 0,5 et 6.

6. Dispositif selon l'une des revendications précédentes, **caractérisé en ce que** le/les espaceurs (12) présentent une forme complémentaire aux éléments d'endoprothèse (10).

7. Dispositif selon l'une des revendications précédentes, **caractérisé en ce qu'**à l'intérieur de la tige (5) et à l'arrière de la ligne axiale (L) occupée par les éléments d'endoprothèse (10) est disposé un matériau de remplissage (7).
